# EUROPÄISCHE PATENTSCHRIFT

(11) **EP 3 320 854 B1**
(45) Veröffentlichungstag und Bekanntmachung des Hinweises auf die Patenterteilung: **08.01.2020**
(21) Anmeldenummer: 16198466.1
(22) Anmeldetag: 11.11.2016
(51) Int. Cl.: A61B 17/04, A61B 17/062, A61B 17/06

(54) **NAHTMATERIAL-HANDHABUNGSVORRICHTUNG ZUM HERSTELLEN EINES NAHTKNOTENS**
SEWING MATERIAL HANDLING DEVICE FOR PRODUCING A SEWN KNOT
DISPOSITIF DE MANIPULATION DE MATÉRIAU DE SUTURE POUR FORMER UN NOEUD DE SUTURE

(43) Veröffentlichungstag der Anmeldung: 16.05.2018
(73) Patentinhaber: INTRASOFT AG, 6331 Hünenberg (CH)
(72) Erfinder: Eid, Dr. med. Karim, 8032 Zürich (CH); Ebner, Benjamin, 79733 Görwihl (DE); Martinoli, Enea, 6600 Locarno (CH); Zuddas, Antonello, 8212 Neuhausen (CH); Hofstetter, Stefan, 6340 Baar (CH); Bastas, Ugur, 6331 Hünenberg (CH)
(74) Vertreter: Patentanwälte Behrmann Wagner PartG mbB

(56) Entgegenhaltungen:
- AT-B- 166 938
- US-A1- 2014 163 583
- US-A1- 2014 296 880

## Beschreibung

Die vorliegende Erfindung betrifft eine Nahtmaterial-Handhabungsvorrichtung nach dem Hauptanspruch. Ferner beschreibt die vorliegende Offenbarung ein System zum Herstellen eines Nahtknotens in einem Nahtträger, wobei ein derartiges System insbesondere eine Nahtmaterial-Handhabungsvorrichtung nach dem Oberbegriff des Hauptanspruchs aufweist.

Gattungsbildende Nahtmaterial-Handhabungsvorrichtungen sind aus dem Stand der Technik allgemein bekannt und werden für zahlreiche Anwendungszwecke eingesetzt, bei welchen ein (üblicherweise aus einem chirurgischen Nahtmaterial bestehender) Faden in eine Naht bzw. einen Knoten überführt werden muss. Eine derartige gattungsbildende Nahtmaterial-Handhabungsvorrichtung ist aus der EP 2 392 265 B1 in Form eines Nähapparats bekannt, welcher einen einen ersten und einen zweiten Backenabschnitt aufweisenden Nähkopf umfasst, der durch einen geeigneten Handabschnitt betätigbar ist. Die Backenabschnitte sind relativ zueinander bewegbar, wobei sich eine solche Anordnung insbesondere eignet, mittels der Backenabschnitte an bzw. auf dem Nahtträger, etwa einen mit der Naht zu versehenden Abschnitt aus einem biologischen Material, zu greifen, um diesen Nahtträger dann mit einer mit dem Faden zu realisierenden Naht zu versehen. Konkret lehrt etwa die EP 2 392 265 B1, dass insbesondere durch manuelle Betätigung die zwei Backenabschnitte auf eine erste bzw. (gegenüberliegende) zweite Flachseite des Nahtträgers greifen können und dann erste Nadelmittel in Form einer biegbaren Nadel von einem ersten der Backenabschnitte durch das Nahtträgermaterial hindurch in den zweiten der Backenabschnitte greifen, dabei den Nahtträger durchstechen und nachfolgend wieder in eine Ausgangslage zurückgezogen werden können. Dabei sieht der beschriebene Stand der Technik vor, dass bei einer der beiden Relativbewegungen geeignete, an den Nadelmitteln vorgesehenem Mitnehmer einen auf einem der Backenabschnitte geführten Faden mitnehmen können, wobei dies bevorzugt mittels eines in einem Nadelende der Nadelmittel ausgebildeten Schlitzes geschieht.

Auf diese Weise ist dann eine Einfachnaht herstellbar, indem durch eine geeignete Bedienperson, etwa einen Operateur, nach dem Greifen des Nahtträgers mittels der bevorzugt zangenartig schwenkbar ausgebildeten Backenabschnitte die Durchstich- und Mitnahmebewegungen der Nadelmittel durchgeführt werden.

Insbesondere jedoch bei komplexeren Anforderungen an das Anbringen eines Nahtmaterial-Fadens an einem (insbesondere biologischen) Nahtträger ist eine derartige Vorgehensweise nicht ausreichend, um eine stabile und insbesondere vor einem unbeabsichtigten Ausreißen geschützte Naht herzustellen. Exemplarisch am Beispiel einer (von einem Knochen abgerissenen) Sehne als biologischem Material, dessen mit Nahtmaterial zu versehendes Sehnenende als Nahtträger verstanden wird, ist eine derartige Vorgehensweise durch Einbringen einer einzelnen Naht oder mehrerer benachbarter Einfachnähte nicht ausreichend, so dass entweder langwierige Heilprozesse ohne weitgehende Nahtunterstützung in Kauf genommen werden müssen oder aber ein Operateur händisch und ohne weitgehende apparative Unterstützung komplexere Knotenmuster - mit dem entsprechenden operativen Aufwand - vorsehen muss.

Aus dem Stand der Technik ist zudem gemäß der US 2014/163583 A1 ein Systeme zur Gewebereparatur bekannt, bei denen eine längliche Gewebereparaturvorrichtung in einen einzigen Schnitt eingeführt werden kann, um auf das beschädigte Gewebe zuzugreifen. Eine Nahtmaterialzuführungsanordnung kann so bemessen sein, dass sie durch einen einzigen Schnitt eingeführt und in der Nähe einer beschädigten oder geplatzten Geweberegion positioniert werden kann.

Aus der AT 166 938 B ist ebenfalls bereits ein chirurgisches Nähinstrument bekannt.
Die US 2014/296880 A1 offenbart zudem eine Nahtmaterialdurchführungs-Vorrichtung bekannt, die eine kerbfreie, rohrförmige Nadel mit einer vorgeformten, gekrümmten Form umfasst. Eine oder mehrere Klampen sind innerhalb der Nadel angeordnet, um die Naht, die von der scharfen distalen Spitze der Nadel erfasst wird, zu sichern und eine weitere Verzweigung der Naht zu verhindern. Die deformierbare Nadel ist in einem Kanal eines Unterkiefers mit einem gekrümmten Führungsweg untergebracht, der sich der gekrümmten Geometrie der vorgeformten Nadel annähert, wodurch die gleichmäßige Rückkehr der Nadel in ihre vorgeformte Form jedes Mal erleichtert wird, wenn die Nadel den Kanal verlässt. Eine Zweinadel-Nahtvorrichtung ist ebenfalls mit einer zweiten kerblosen Nadel versehen, um einen Matratzenschnitt zu ermöglichen. Verfahren zum Laden eines Fadens auf eine kerblose Nadel in einem Nahtpasser sind ebenfalls vorgesehen.

Aufgabe der vorliegenden Erfindung ist es daher, eine Nahtmaterial-Handhabungsvorrichtung im Hinblick auf die Unterstützung komplexerer Naht- und Knotenstrukturen, welche durch eine solche Vorrichtung erzeugt bzw. unterstützt werden können, zu verbessern, dabei insbesondere das Einbringen einer komplexeren Naht- bzw. Knotenstruktur zu ermöglichen, welche im Hinblick auf einen Halt am Nahtträger, eine Gefahr eines unbeabsichtigten Ab- bzw. Ausreißens sowie eine mechanische Festigkeit verbessert ist. Dabei ist zwar eine Befestigung des Fadens an bzw. in einem aus biologischem Material realisierten Nahtträger bevorzugt, soll jedoch das Anwendungsgebiet der Erfindung nicht beschränken.

Die Aufgabe wird durch die Nahtmaterial-Handhabungsvorrichtung mit den Merkmalen des Hauptanspruchs gelöst. Vorteilhafte Weiterbildungen der Erfindung sind in den jeweiligen Unteransprüchen beschrieben, wobei strukturelle Weiterbildungen der jeweiligen Erfindungsgegenstände auch für den jeweils anderen Erfindungsgegenstand als zur Erfindung gehörig offenbart gelten sollen. Auch sollen im Rahmen der vorliegenden Offenbarung Verfahrensaspekte als offenbart gelten, welche sich insbesondere aus einer Handhabung bzw. einem Betrieb der erfindungsgemäßen Nahtmaterial-Handhabungsvorrichtung sowie einer Handhabung bzw. einem Betrieb des Systems zum Herstellen eines Nahtknotens in einem Nahtträger ergeben, so dass auch ein derartiges Betriebsverfahren als offenbart gelten soll.

In erfindungsgemäß vorteilhafter Weise ordnet die erfindungsgemäße Nahtmaterial-Handhabungsvorrichtung den ersten Nadelmitteln zusätzlich zweite Nadelmittel diesen benachbart zu, wobei diese im Hinblick auf ihre Funktionalität gegenüber den (aus dem gattungsbildenden Stand der Technik bekannten) ersten Nadelmitteln erweitert sind: Diese sind nämlich nicht nur ausgebildet, einen zweiten Durchstich- sowie Fadenmitnahmevorgang für ein dem ersten Fadenende entgegengesetztes zweites Fadenende des (einzelnen) Fadens herzustellen, wenn die Backenabschnitte geeignet geschlossen sind bzw. sich in der aufeinander bewegten Relativposition befinden. Auch sind die zweiten Nadelmittel erfindungsgemäß vorgesehen, ein zweifaches Durchstechen des Nahtträges durchzuführen, so dass, in Verbindung mit der Funktionalität der ersten Nadelmittel, insgesamt (mindestens) drei Durchstich-Vorgänge am Nahtträger, einander benachbart, durchgeführt werden können. Zu diesem Zweck weisen die zweiten Nadelmittel einen Mitnehmerabschnitt auf, welcher das zweite Fadenende nicht nur zum Durchstechen für die aufeinanderfolgenden beiden Durchstichvorgänge mitnimmt, auch ist dieser erfindungsgemäße Mitnehmerabschnitt ausgebildet, eine Schwenk- bzw. Bogenbewegung des zweiten Fadenendes, insbesondere zwischen den aufeinanderfolgenden Durchstichvorgängen, durchzuführen.

Damit gestattet es dann die Erfindung, nicht nur eine Mehrzahl von Durchstichvorgängen am bzw. im Nahtträger auszuführen, auch wird durch die erfindungsgemäße Schwenk- bzw. Bogenbewegung, realisiert durch den geeignet schwenkbar ausgebildeten Mitnehmerabschnitt, eine (dann geeignet straffbare bzw. zu straffende) Querverbindung auf der betreffenden Oberfläche des Nahtträgers ausgebildet, welche, neben den eigentlichen Durchstichen, zusätzliche Festigkeit, verbunden mit Materialschonung, ermöglicht und so die erfindungsgemäße Aufgabe einer festen, gleichzeitig ausrißsicheren und schonenden Naht (Nahtknoten bzw. Knoten) ermöglicht.

Als wesentliche mechanisch-konstruktive Komponente der Erfindung zum Ausgestalten des erfindungsgemäßen Mitnehmerabschnitts (Handhabungsvorrichtung) bzw. der Fadenführungs- und entsprechenden Fadenumlenkmittel (System) ist vorgesehen, dass die zweiten Nadelmittel einen bevorzugt kreisbogenförmig gebogenen und endseitig einen Nadelabschnitt sowie den Mitnehmerabschnitt zum lösbaren Einsetzen des zweiten Fadenendes ausgebildeten Bogenschaft aufweisen. Dabei kann dieser Nadelabschnitt insbesondere auch durch das zweite Fadenende selbst realisiert sein, indem nämlich ein geeignet an diesem zweiten Ende mit einer (bevorzugt starren) Nadel versehenes Fadenende in den weiterbildungsgemäßen Mitnehmerabschnitt eingesetzt wird und so den Nadelabschnitt der zweiten Nadelmittel realisiert.

Diese zweiten Nadelmittel sind dann erfindungsgemäß zusätzlich weiterbildend endseitig einer bevorzugt manuell drehbar und am Nähkopf gelagerten Schaftbaugruppe vorgesehen, wobei in einer besonders bevorzugten Ausführungsform dieses drehbare Festlegen in einem der Backenabschnitte erfolgt. Über den geeignet dem Nähkopf zugeordneten Handhabungsabschnitt kann dann in ansonsten bekannter Weise, etwa mittels eines geeignet betätigbaren Hebels, diese Schaftbaugruppe in Rotation (Hin- sowie Rückdrehung) versetzt werden, um den erfindungsgemäßen Betrieb der zweiten Nadelmittel (erstes Durchstechen, Schwenk- bzw. Bogenbewegung und, im Hinblick auf die zu durchstechenden Flächen des Nahtträgers, entgegengesetztes zweites Durchstechen, dann Rückbewegung) zu bewirken.

Zusätzlich konstruktiv vorteilhaft ist es, diese Schaftbaugruppe an einem Übergangsabschnitt zu den zweiten Nadelmitteln exzentrisch ausgelenkt bzw. gekröpft auszubilden, so dass insoweit elegant und mit geringem mechanischkonstruktiven Aufwand die erfindungsgemäße Schwenk- bzw. Rotationsbewegung des zweiten Fadenendes gewährleistet ist.

Während es zudem weiterbildend besonders bevorzugt ist, einem der Backenabschnitte eine Führung und Aufnahme für das erste Fadenende zuzuordnen (welche gemäß einer bevorzugten Realisierungsform im Rahmen des Systems schlaufenartig ausgestaltet ist und etwa, analog zum Vorgehen gemäß EP 2 392 265 B1, das Mitnehmen durch die ersten Nadelmittel in Verbindung mit einem durch diese Nadelmittel durchzuführenden Durchstichvorgang vorsieht), ist es im Rahmen der Erfindung günstig, benachbart dieser Führung bzw. Aufnahme des ersten Fadenendes die weiterbildungsgemäßen Fadenendeaufnehmermittel für das zweite Fadenende vorzusehen. Dies führt dann in der erfindungsgemäßen Realisierung dazu, dass nach dem erfindungsgemäßen ersten Durchstichbetrieb, dem Schwenk- bzw. Bogenvorgang und zweiten Durchstichbetrieb, jeweils des zweiten Fadenendes, dieses geeignet an bzw. in dem Fadenendeaufnehmermittel abgelegt (weiter bevorzugt eingerastet bzw. eingeschnappt) werden kann, so dass dann die zweiten Nadelmittel ohne ansitzendes zweites Fadenende zurückbewegt werden können. Diese besonders bevorzugte Ausführungsform führt dann dazu, dass bei einem nachfolgenden Lösen der Backenabschnitte vom Nahtträger und einem Entfernen des Nähkopfes vom Nahtträger eine Zugkraft in das zweite Fadenende über diese Fadenendeaufnehmermittel eingebracht wird, was dann das Straffen des Fadens und mithin das Fertigstellen der komplexen Naht- bzw. Knotenstruktur ermöglicht.

Dabei ist der Begriff der Flachseite des Nahtträgers breit auszulegen und insbesondere nicht auf eine plane oder ebene Seite beschränkt, vielmehr gilt als ordnungsgemäße Flachseite auch derjenige Oberflächenabschnitt des Nahtträgers, der - verformt oder unverformt - das Einbringen der erfindungsgemäßen Durchstiche gestattet.

Es wird deutlich, dass im eleganten Zusammenspiel zwischen dem Paar der Backenabschnitte und der daran bzw. damit realisierten Führung und Bewegung der ersten und zweiten Nadelmittel diese für das Herstellen der komplexen Knotenstruktur notwendigen Vorgänge wirksam durchgeführt bzw. unterstützt werden können; so sorgt etwa ein weiterbildungsgemäß vorgesehener Gleitabschnitt an dem den zweiten Nadelmitteln gegenüberliegenden Backenabschnitt dafür, dass die Schwenk- bzw. Bogenbewegung der zweiten Nadelmittel bestmöglich unterstützt wird ohne dass, etwa in einem operativen Betrieb, umliegendes Gewebe beschädigt wird oder andere Nachteile entstehen. Im Ergebnis gestattet es damit die Erfindung, in überraschend einfacher und eleganter Weise eine komplexe Nahtstruktur an bzw. in einem (bevorzugt biologischen) Nahtträger vorzusehen, welche drei einander benachbarte Durchstiche sowie zwei stegartig gestraffte und diese Durchstiche, bevorzugt an einander gegenüberliegenden Seiten, verbindende Querschlaufen vorsieht. Das Ergebnis ist eine hochgradig stabile, gewebefreundliche Nahtstruktur (Knotenstruktur), welche der aus der medizinischen Literatur bekannten sogenannten Mason-Allen - Nahtstruktur ähnelt, ohne das diese jedoch aufwendig durch einen entsprechend geschulten Operateur rein manuell durchgeführt werden muss. Offensichtlich sind die sich daraus für die medizinische Praxis (aber nicht nur für diese) ergebenden Vorteile einer Zeitersparnis, einer Absenkung des Schulungs- und Trainingsaufwandes für Operateure und Erhöhung der Qualität Ergebnis der erzeugten Naht, so dass die vorliegende Erfindung das Potential birgt, in bislang ungeahnter Weise komplexe, mechanisch stabile und gewebefreundliche Nahtstrukturen zur Verbindung eines Nahtmaterial-Fadens mit einem Nahtträger einzusetzen. Nicht zuletzt besitzt die vorliegende Erfindung Vorteile für endoskopische Operationsverfahren sowie etwa für die Athroskopie.

Während zudem die vorliegende Erfindung besonders bevorzugt als medizinisches bzw. chirurgisches Instrument realisiert ist und in geeigneter Weise zur (bevorzugt) manuellen Betätigung mit einem Handhabungs- bzw. Bedienabschnitt am Nähkopf versehen ist, ist gleichwohl die vorliegende Erfindung nicht auf diesen Anwendungsfall beschränkt. Vielmehr bietet es sich sowohl an, im Hinblick auf etwa mikroinvasive Anwendungen eine derartige Vorrichtung zu nutzen bzw. geometrisch weiter zu verkleinern (und insbesondere dann auch über ferngesteuerte bzw. endoskopische Aggregate zu betätigen bzw. anzusteuern), als auch alternativ es im Grundsatz von Weiterbildungen der Erfindung umfasst ist, diese für textile, pflanzliche, aus Kunststoff realisierte oder andere Nahtträger auszugestalten, bei welchen gleichermaßen die diskutierten Vorteile der resultierbaren Naht einfach realisierbar werden.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
- Fig. 1, Fig. 2: Perspektivansichten der Nahtmaterial-Handhabungsvorrichtung gemäß einem ersten Ausführungsbeispiel der Erfindung im geöffneten, d.h. nicht an einer Eingriffsposition an einem Nahtträger befindlichen Position, mit eingelegtem Faden;
- Fig. 3: eine schematische Explosionsdarstellung eines Backenabschnitts sowie der zweiten Nadelmittel gemäß Ausführungsbeispiel der Figuren 1, 2 mit schematisch dazu in Bezug gesetztem Faden;
- Fig. 4: eine Detail-Schnittansicht des unteren Backenabschnitts im Ausführungsbeispiel der Figuren 1 bis 3 mit darin geführten ersten Nadelmitteln;
- Fig. 5: mit Teildarstellungen (a) bis (e) Fertigungsschritte zum Herstellen einer komplexen Naht bzw. eines komplexen Nahtknotens aus dem Faden durch Handbetätigung der Nahtmaterial-Handhabungsvorrichtung nach einem der Ansprüche 1 bis 4, insoweit entsprechend der Funktionalität eines Ausführungsbeispiels des Systems;
- Fig. 6: in Teilbildern (a) und (b) Ansichten einer fertiggestellten Naht nach einem Entfernen der Nahtmaterial-Handhabungsvorrichtung von einer Eingriffsposition am Nahtträger;
- Fig. 7: mit Teildarstellungen (a) bis (c) Details einer Fadenhandhabung nach dem Fertigen der Durchstiche,
- Fig. 8: insoweit entsprechend einer Detaillierung der Funktionsfähigkeit der Nahtmaterial-Handhabungsvorrichtung bzw. des Systems zwischen den Verfahrensschritten (c) und (d) in Figur 5 und eine Detail-Querschnittsdarstellung durch die Nahtmaterial-Handhabungsvorrichtung bzw. das System im gezeigten Ausführungsbeispiel beim Betreiben der zweiten Nadelmittel nach erfolgter Rotation derselben.

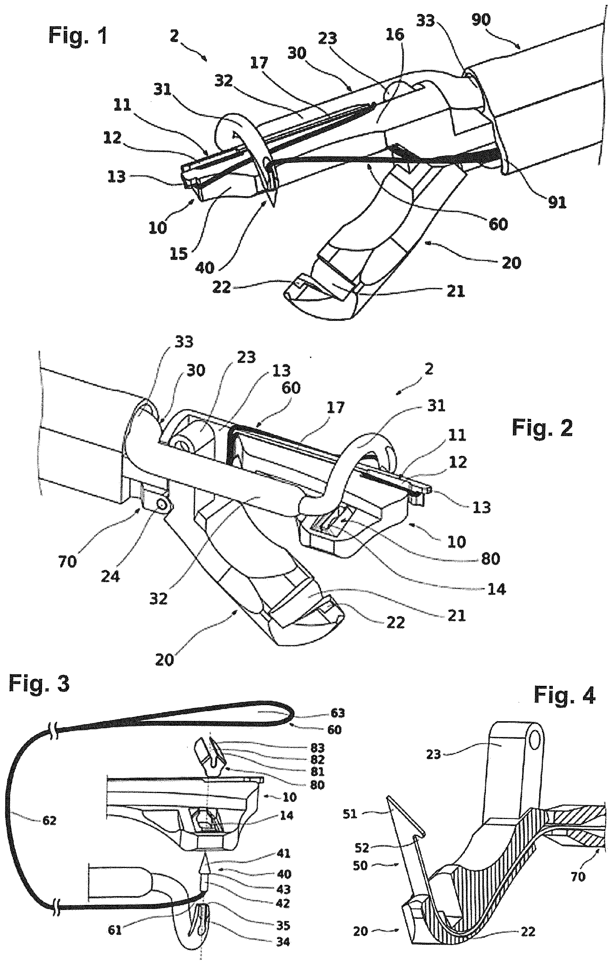

Die Darstelllungen der Figuren 1 bis 4 illustrieren die mechanisch-strukturelle Realisierung eines Nähkopfs im Rahmen der gezeigten Nahtmaterial-Handhabungsvorrichtung bzw. des damit realisierten Systems dieses ersten Ausführungsbeispiels. Ein Paar von Backenabschnitten bestehend aus einem unteren Backenabschnitt 20 und einem oberen Backenabschnitt 10 ist bis mittels eines Schwenkgelenks 24 gegeneinander schwenkbar miteinander zangenartig verbunden. Die Darstellungen der Figur 1, Figur 2 zeigen insoweit eine geöffnete Position dieser Backenabschnitte, wobei diese Backenabschnitte dann zum Zusammenwirken mit einem hier als schematischer Muskelabschnitt 110 gezeigten Nahtträger in einer Eingriffsposition dergestalt gebracht werden können, dass sowohl der obere Backenabschnitt 10, als auch der untere Backenabschnitt 20 auf einer entsprechenden oberen bzw. unteren Flachseite dieses Nahtträgers liegen können. Zu diesem Zweck bewegen sich die Backenabschnitte relativ zueinander, wobei hier insbesondere der untere Backenabschnitt 20 durch nicht näher im Detail gezeigte Betätigungsmittel, auf einen Backenarm 23 wirkend, diese schließende Schwenkbewebung ausführen können. Geeignete Betätigungsmittel sind in einem lediglich schematisch gezeigten Betätigungs- bzw. Handabschnitt 90 dem Backenabschnitt 20 zugeordnet und in einem Gehäuse bzw. unter einer Abdeckung 91 geführt.

Der hier mittels einer Baugruppe 70 schwenkbar dargestellte untere Backenabschnitt 20, vergleiche insbesondere die im Längsschnitt dargestellte Detailansicht der Figur 4, weist einen sich entlang der Erstreckung des Backenabschnitts erstreckenden Kanal 22 auf, welcher in Richtung auf ein eingriffsseitiges (frontseitigtes bzw. distales) Ende aufwärts gebogen ist, so dass eine in diesem Kanal 22 geführte Flachnadel 50 aus diesem eingriffseitigen Ende nach oben, d.h. in Richtung auf den gegenüberliegenden zweiten Backenabschnitt 10, austreten kann.

Prinzipiell realisiert auf diese Weise der untere Backenabschnitt mit der darin vorgesehenen gebogenen bzw. gekrümmten Führung 22 für die biegbare Flachnadel 50 eine der EP 2 392 265 B1 zum Stand der Technik beschriebene Lehre, nämlich die Möglichkeit, die Nadel 50 mit ihrem spitz zulaufenden Nadelende 51 in nachfolgend im Detail zu beschreibender Weise aus der Eingriffsposition des unteren Backenabschnitts 10 von unten durch den Nahtträger (und wieder zurück) zu führen; diese Bewegung der Nadel geschieht mit Hilfe einer nicht dargestellten, am rückwärtigen (proximalen) Ende des Backenabschnitt vorgesehenen Betätigungseinheit, die, etwa die Nadel 50 klemmend, eine das Nadelende 51 bewegende Schub- und Zugbewegung auf die Nadel 50 ausüben kann. Zusätzlich weist die Nadel 50 einen Vorsprungs- bzw. Hakenabschnitt 52 auf, welcher in nachfolgend zu beschreibender Weise als Mitnehmer zum Aufnehmen und Mitnehmen eines Fadenendes (hier des ersten Fadenendes 63) des in Figur 3 gezeigten Fadens 60 dient.

Der in den Figuren 1 bis 3 gezeigte obere Backenabschnitt 10 weist einen langgestreckten Träger 16 auf, an welchem im Ausgangszustand, nämlich bei noch nicht auf bzw. in den Nahtträger eingebrachtem Faden 60, dieser Faden gehalten ist. Genauer gesagt erstreckt sich in diesem Vorbereitungszustand der Vorrichtung der Faden 60 mit seinem ersten, schlaufenförmig ausgebildeten Ende 63 von einem diesem zugeordneten Führungsabschnitt 17 des Trägers 16, wird an einem proximalen (bezogen auf den Nahtträger rückwärtigen) Ende abwärts geführt und dann mit seinem dem ersten Ende 63 gegenüberliegenden zweiten Fadenende 40 von einer zweiten Nadeleinheit (Nadelmittel) 30 aufgenommen, die, anschließend an einen gekröpften Armabschnitt 32, endseitig in einem Bogenschaft 31 mündet, welcher wiederum an seinem äußeren Ende 35 einen Aufnahmeschlitz 34 zum Einsetzen des zweiten Fadenendes 40 aufweist. Genauer gesagt ist das zweite Fadenende 40 so ausgestaltet, dass der endseitige Fadenabschnitt 61 des Fadens 60 über einen zylindrischen Schaftabschnitt 42, angepasst an den Schlitz 34, in eine Nadelspitze 41 mündet, so dass im eingesteckten Zustand des zweiten Nadelendes 40 in das vordere Ende 35 der zweiten Nadelmittel 30 diese endseitig zum Durchführen weiterer Durchstichvorgänge des Nahtträgers 110 ausgebildet sind, wie gleichermaßen nachfolgend zu erläutern sein wird.

Wie sich insbesondere aus den Darstellungen der Figur 1, Figur 2 ergibt, ist der gekröpfte Arm- bzw. Schaftabschnitt 32 der zweiten Nadelmittel 30 in Richtung auf die Handhabungs- bzw. Bedieneinheit 90 in der Art einer darin drehbar gelagerten Welle 33 ausgebildet, wobei, noch weiter rückwärtig und in den Figuren nicht gezeigt, dann dieser Welle Handhabungs- bzw. Drehmittel so zugeordnet sind, dass eine Drehung um die Drehachse 33 bewirkt werden kann, damit ein Verschwenken des gekröpften Schaft- bzw. Armabschnitts sowie des daran distal vorgesehenen Bogenschaft 31 mit endseitiger Nadel 41 bewirkt wird. Erkennbar ist zudem, dass dieser Dreh- bzw. Schwenkvorgang um den Trägerabschnitt 16 des oberen Backenabschnitts 10 erfolgt.

Durch diese Struktur ist es damit ermöglicht, dass bei Drehung um die Welle 33 der Bogenschaft 31 (zunächst mit darin gehaltener Nadel 40) eine schwenk- bzw. bogenartige Bewegung durchführt, wobei typischerweise bei diesem Betriebszustand die Backenabschnitte zueinander bewegt und damit geschlossen sind. In diesem Betriebszustand steht dann ein im unteren Backenabschnitt 20 vorgesehener und quer zur Erstreckungsrichtung (und damit quer zur Erstreckungsrichtung des langgestreckten Trägers 16) verlaufender Führungsschlitz 21 im Eingriff mit der Nadelspitze 41, sodass diese an bzw. im Schlitz 21 abgleiten kann und insbesondere dadurch wirksam eine Beeinträchtigung umgebenden Gewebes oder dergleichen an einem medizinischen Eingriffsort vermieden ist. Zusätzlich erleichtert der Schlitz 21 das Erzeugen des erneuten Durchstichs.

Insbesondere die Ansichten der Figuren 2 und 3 zeigen zusätzlich in Form der Baugruppe 80 einen Nadel- bzw. Fadenmitnehmer (Fadenendeaufnehmermittel), welche in einen Durchbruch 14 des oberen Backenabschnitts 10 eingesetzt sind und ein Paar gegeneinander bewegbare Schenkel 81 aufweisen, die, jeweilige Kanten 83 ausbildend, einander an einer Schlitzöffnung 82 gegenüberliegend angeordnet sind. Die Baugruppe 80, geeignet angepasst an das Nadelende 41 bzw. den zugehörigen Nadelschaft 42, ist ausgebildet, dieses Nadelende schnappend zu verrasten, sobald am Ende der von der Baugruppe 30 durchgeführten Schwenkbewegung das am vorderen (distalen) Ende des Bogenarms 31 sitzende Nadelende (hier von unten) den Backenabschnitt 10 erreicht und in die Baugruppe 80 eintritt. Durch Verschnappen der Schenkelabschnitte 81 mit dem zwischen der Nadelspitze 41 und dem Schaft 42 des zweiten Fadenendes gebildeten Absatzes gestattet es dann eine rückziehende (entgegensetzte) Schwenkbewegung der Baugruppe 30, dieses Nadelende vom Bogenschaft 31 abzuziehen, so dass es in der durch die Baugruppe 80 gebildeten Aufnahme im oberen Backenabschnitt 10 verbleibt.

In etwa benachbart diesem Abschnitt 14 im oberen Backenabschnitt 10 ist in diesem randseitig eine Öffnung 12 ausgebildet, welche, als Teil der das erste (schlaufenförmige) Fadenende 63 haltenden Führung 11, schlitzartig und zum Durchgreifen bzw. Zusammenwirken mit dem Nadelende 51 der ersten Flachnadelmittel 50 ausgebildet ist. Genauer gesagt zeigen die Darstellungen der Figur 1, Figur 2 den Betriebszustand des an der Baugruppe 11 gehaltenen Fadens (genauer: Der Fadenschlaufe 63 als erstes Fadenende), wobei diesbezüglich zusätzlich ein endseitiger Vorsprung 13 ein Abgleiten dieser Fadenschlaufe nach oben verhindert. Im Zusammenwirken der ersten Flachnadelmittel 50 mit der Fadenaufnehmerbaugruppe 11 und bei geschlossenen Backenabschnitten 10, 20 ist die Nadel 50 in der Lage, mit ihrem eingriffseitigen Nadelende 51 durch den Öffnungsschlitz 12 von unten nach oben zu greifen, bewegt dabei einen Abschnitt der Fadenschlaufe 63 über das dreieckförmige Spitzenende 51 (Figur 4), so dass bei einem nachfolgenden Herausziehen der Nadel 50 durch Zugbetätigung der Schiebereinheit 70 (insoweit entsprechend einer Abwärtsbewegung des Nadelabschnitts 51) der seitliche Haken- bzw. Vorsprungsabschnitt 52 die Schlaufe 63 mitnehmen und insoweit dann auch einen Fadentransport durch den Nahtträger 110 bewirken kann. Im dann zurückgezogenen Zustand der Nadel 50 im Backenabschnitt 20 sorgt der Haken 52 (etwa gegenüber einem theoretisch denkbaren Schlitz) zudem für eine günstige Halte- bzw. Klemmwirkung des mitgenommenen Fadens im Backenabschnitt.

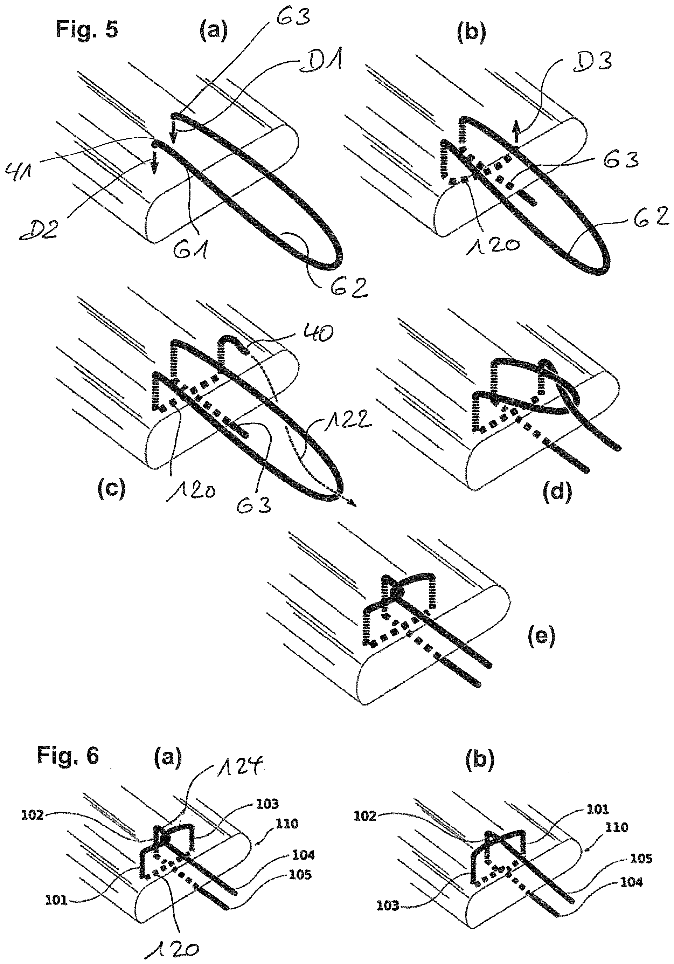

Die Darstellungen der Figuren 5 und 6 führen diese mechanische Funktionalität und Struktur der Nahtmaterial-Handhabungsvorrichtung (genauer: des Nähkopfes der Figuren 1 bis 3) zusammen mit den Bewegungen, welche der Faden 60 bei diesen Handhabungsschritten ausführt, insoweit beschreibt also die Figur 5 den Betrieb bzw. die Wirksamkeit des Systems im Zusammenwirken zwischen Nahtmaterial-Handhabungsvorrichtung und darin zunächst aufgenommenem und später auf bzw. in den Nahtträger 110 gebrachtem Faden 60.

Die in Figur 5 schematisch gezeigte Abfolge der Handhabungsschritte des Fadens 60 bedingt zunächst, dass in vorbeschriebener Weise der obere Backenabschnitt 10 und der untere Backenabschnitt 20 von oben (10) bzw. von unten (20) auf das in der Figur 5 gezeigte Gewebeende 110 als Nahtträger greifen. Aus dieser Position heraus erfolgt daher zunächst das Einbringen von zwei Durchstichen D1 und D2 mit jeweils zugehörigen Fadenenden 63 (für D1) bzw. 41 (für D2) . Das Teilbild in Figur 5 (a) ordnet diesen Durchstichvorgängen jeweils Pfeile zu, wobei, in der vorbeschriebenen Weise, der Durchstich D1 durch den Nahtträger 110 von seiner in den Figuren oben gezeigten Oberseite (insoweit entsprechend der Seite des oberen Backenabschnitts 10) durch das Trägermaterial hindurch bis zu dessen Unterseite erfolgt. Dabei wird durch Wirkung der ersten Nadelmittel (Flachnadel 50) zunächst aus Richtung des unteren Backenabschnitts 20 das Trägermaterial 110 von unten nach oben durchstochen, in der vorbeschriebenen Weise greift dann der Hakenabschnitt 52 der Nadel 50 das Fadenende 63 und zieht dieses entsprechend der Markierung für den Durchstich D1 abwärts.

Zum Herstellen des zweiten Durchstichs D2 bewegen sich die zweiten Nadelmittel 30 mit ihrem vorderen Bogenschaft 31 und darin mittels Nadelende 41 sitzender zweiten Fadenende aus der in Figur 1, Figur 2 gezeigten Position und durchstechen wiederum von oben nach unten das Trägermaterial des Nahtträgers 110, nehmen dabei das Nadelende 41 mit. Am Ende dieses Vorgangs entsteht die in Figur 5 (a) gezeigte Schlaufe, welche im Wesentlichen von einem das erste Fadenende 63 und das zweite Fadenende 40 verbindenden Faden-Zwischenabschnitt 62 gebildet wird. Es kommt auf die Reihenfolge dieser Durchstiche nicht an, vielmehr kann der Durchstich D1 auch nach dem Durchstich D2, sogar nach dem nachfolgend noch zu beschreibenden Durchstich D3, erfolgen.

Ein weiteres Verschwenken der zweiten Nadelmittelbaugruppe 30 mit endseitigem Bogenschaft 31 (bei nach wie vor darin sitzender Nadel 41) führt dazu, dass das zweite Nahtende auf der Unterseite des Nahtträgers einer stegartigen und querverlaufenden Nahtabschnitt 120 (gestrichelt gezeigt) ausführt, und zwar bis, beim weiteren (rotatorischen) Verschwenken des Bogenschafts 31, dessen Ende, d.h. die darin sitzende Nadel 41 als zweites Fadenende wiederum die untere Oberfläche an der unteren Flachseite des Nahtträgers erreicht. Ein weiteres Drehen bzw. Verschwenken erzeugt dann entsprechend Pfeildarstellung D3 (Teilbild Figur 5 (b)) einen dritten Durchstich durch den Träger 110, diesmal aufwärts gerichtet, so dass am Ende dieses Durchstichs das Fadenende 40 (bzw. die dieses realisierende Nadel 41) in Aufwärtsrichtung und aus der Oberseite des Nahtträgers 110 austritt. Die Teildarstellung der Figur 5 (b) verdeutlicht, wie das erste Fadenende (Schlaufe 63) zum freien Ende des Nahtträgers 110 weist, dieses ist gleichermaßen gestrichelt dargestellt, da unterhalb dieses Trägers verlaufend.

Am Ende des Durchstichs D3, Teilfigur (b), erreicht zudem, wie vorstehend beschrieben, die Nadelspitze 41 samt Schaft 42 die als Fadenendeaufnehmermittel wirkende Baugruppe 80. Genauer gesagt durchgreift die Nadelspitze 41 die zwischen dem Backenpaar 81 gebildete schlitzartige Öffnung 82 und verrastet darin, wenn die nunmehr erfolgende entgegengesetzte Rotations- bzw. Drehbewegung der zweiten Nadelmittel 30 (samt des damit bewegten Bogenabschnitts 41) entgegen der Pfeilrichtung gemäß D3 bewegt wird. Dies führt dazu, dass das zweite Fadenende 40 mit ansitzender Nadel 41 außer Eingriff mit dem schlitzförmigen Aufnahmebereich 34 des Bogenschafts 31 gerät und damit die Nadelmittel 30 das zweite Fadenende 40 an die Mitnehmermittel 80 im frontseitigen Ende 14 des oberen Backenabschnitts 10 übergeben.

Zum Zeitpunkt des Betätigungsvorgangs gemäß Teilbild (c) in Figur 5 löst die die Nahtmaterial-Handhabungsvorrichtung bedienende Person (also z. B. der Operateur) das Backenpaar 10, 20 aus ihrer berührenden Eingriffsposition am Träger 110, so dass mit dem Entfernen der Handhabungsvorrichtung vom Träger gleichermaßen eine Zugkraft auf die Nahtenden 63, 41 ausgeübt und, in nachfolgend zu beschreibender Weise, dann die komplexe Naht (Knotenanordnung) gestrafft werden kann.

Dabei führt die vorbeschriebene Führung des Fadens 60 insbesondere auf dem oberen Backenabschnitt 10 dazu, dass bei diesem Lösen des Backenpaares vom Nahtträger und einem damit verbundenen Abstreifen des Fadens vom Nähkopf das Nahtende 40 in der in Teilfigur (c) gezeigten Weise durch die im Wesentlichen vom Faden-Verbindungsabschnitt 62 gebildete Schlaufe entlang der Pfeilrichtung 122 geführt wird und entsprechend der weitere Straffungszustände des Fadens darstellenden Teilbilder (d) und (e) zu einer Verknotung bzw. einer oberen Steg- und Schlaufenbildung auf dem Träger 110 führt. Ergebnis ist das komplexe Knotenbild der Naht gemäß Figur 6 (a), wobei der Fadenabschnitt 102 dem ersten Durchstich D1, der Fadenabschnitt 101 dem zweiten Durchstich D2 und der Fadenabschnitt 103 dem dritten Durchstich D3 entspricht. Der gestraffte Schlaufenabschnitt 120 ist zu einer festen, die Abschnitte 101 und 103 unterseitig des Trägers 110 verbindenden Querstegstruktur geworden, während der Mittelabschnitt 62 zu einer umgelenkten oberen Quersteg- und Knotenstruktur 124 auf der gegenüberliegenden Oberseite des Trägers geworden ist. Das gezeigte Fadenende 105 führt zum Schlaufenabschnitt 63 als erstem Fadenende, Fadenabschnitt 104 zum zweiten Fadenende 40 (dargestellt ohne Nadel 41).

Der so entstehende Knoten, ähnlich der aus der medizinischen Literatur bekannten Mason-Allen-Nahtstruktur, stellt im Hinblick auf Reißfestigkeit und Belastung des Nahtträgers, insbesondere bei biologisch-medizinischen Anwendungen, ein Optimum zwischen Ausrissfestigkeit und Trägerbelastung dar und eignet sich dabei insbesondere für Anwendungen im Zusammenhang mit dem Befestigen von Nahtmaterial an Sehnen-, Band- oder Muskelenden oder dergleichen stark belasteten Abschnitten.

Gegenüber der Darstellung der Figur 6 (a) stellt die Teilfigur (b) eine gegenüberliegende Flachseite des Nahtträgers dar, also gegenüber der Darstellung der Figur 6 (a) um 180 Grad gedreht.

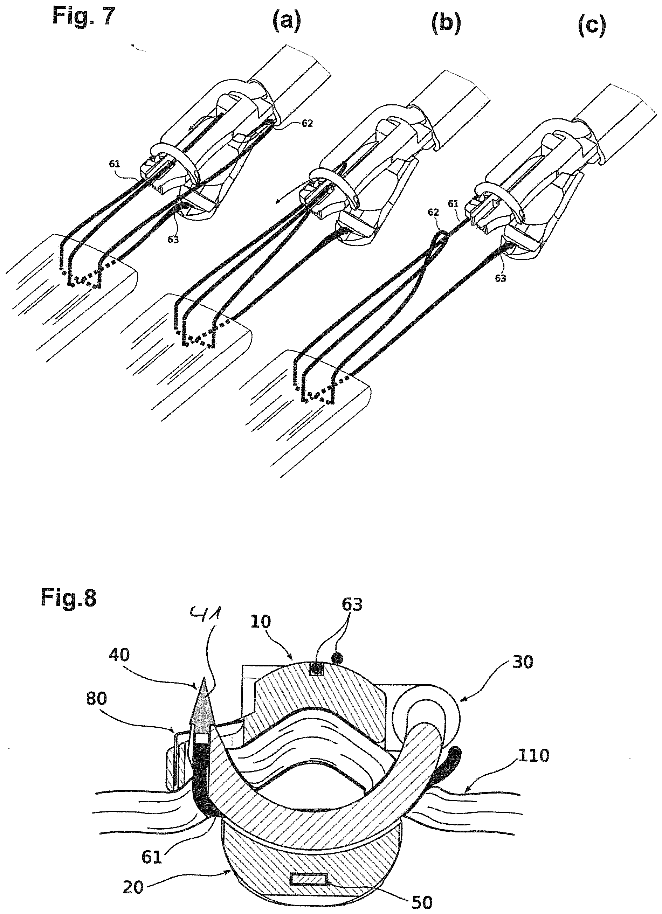

Die Detaildarstellungen der Figuren 7 bzw. 8 erläutern zusätzlich konstruktive bzw. handhabungsmäßige Details der vorbeschriebenen Realisierung der erfindungsgemäßen Nahtmaterial-Handhabungsvorrichtung bzw. des damit realisierten Systems. So wird insbesondere anhand der Teilfiguren (a) bis (c) deutlich, wie im Verlauf des Abziehens des Nähkopfes mit dem Paar von Backenabschnitten 10, 20 (insoweit nach dem Herstellen des Zustands gemäß Teilbild (b) in Figur 5) die Fadenschlaufe im Verbindungsabschnitt 62 von ihrer Halte- bzw. Führungsposition auf dem oberen Backenabschnitt abgleitet und in die Führung gemäß Teilbild (c) von Figur 5 geführt wird. Es wird insbesondere deutlich, dass das erste Fadenende (Schlaufe 63) nach dem Durchführen des zugehörigen ersten Durchstichs D1 im unteren Backenabschnitt 10 gehalten ist und von diesem mitgenommen wird, während das zweite Fadenende 40 (mit endseitiger Nadel 41 und zugehörigem endseitigem Fadenabschnitt 61) in den Aufnahme- und Mitnehmermitteln 80 gehalten ist. Der Verbindungsabschnitt 62 gleitet, wie insbesondere aus einem Vergleich der Teildarstellungen (a) bis (c) von Figur 7 erkennbar ist, zwischen einer oberen Fläche des Trägerabschnitts 16 des oberen Backenabschnitts und dem gekröpften Arm 32 der schwenkbaren Nadelmittel 30 ab, bis die in Teilfigur (c) von Figur 7 gezeigte Schlaufenbildung (insoweit analog Figur 5 (c)), entsteht, die dann im Hinblick auf die Teildarstellungen (d) und (e) von Figur 5 durch weiteres Ziehen des Kopfes weg vom Träger 110 gestrafft werden kann.

Die Figur 8 verdeutlicht zusätzlich im Detail das Zusammenwirken der Baugruppen, wobei die Darstellung der Figur 8 denjenigen Betriebszustand (insoweit analog Figur 5 (b)) beschreibt, bei welchem der Bogenschaft 31 den Träger 110 für den dritten Durchstich erneut durchstoßen und das zweite Fadenende mit frontseitiger Nadelspitze 41 in die Mitnehmereinheit 80 gebraucht hat, an welcher die Nadel 41 verastet ist.

Die vorliegende Erfindung ist nicht auf die Details des in den Figuren 1 bis 8 gezeigten ersten Ausführungsbeispiels beschränkt. Vielmehr ist sowohl, wie bereits dargelegt, die Abfolge der Durchstiche D1 bis D3 lediglich bevorzugt, kann insbesondere im Hinblick auf die Funktionalität der ersten Nadelmittel und den Zeitpunkt der Betätigung dieser ersten Nadelmittel variiert werden. Gleichermaßen ist auch die Zuordnung der Funktionsaggregate zu den oberen bzw. unteren Backenabschnitten nicht festgelegt und kann auch umgekehrt verlaufen, ebenso wie etwa die Funktionalität der ersten Nadelmittel umgekehrt sein kann (d.h. das erste Fadenende wird von unten zugeführt statt von oben mitgenommen), ebenso wie etwa die Funktionalität der zweiten Nadelmittel umgekehrt sein kann (d.h. das zweite Fadenende wird erst nach erfolgtem Durchstechen mitgenommen und dann beim rückwärtigen Verschwenken mit zurückgezogen statt, wie vorbeschrieben, mit dem Durchstechen durch den Träger bis zur Mitnehmeraufnahme gebracht). Auch sieht eine mögliche alternative Ausgestaltung der Erfindung vor, dass die im unteren Backenabschnitt vorgesehene biegbare Flachnadel 50 nicht etwa bewegbar in diesem unteren Backenabschnitt geführt ist, sondern starr mit dem unteren **(oder oberen)** Backenabschnitt verbunden ist. Insoweit würde dann diese mit ihrem Nadelende heraus- bzw. hervorragende starre Nadel zusammen mit der Bewegung des (unteren **bzw. oberen**) Backenabschnitts bewegt werden und entsprechend betätigbar sein.

## Patentansprüche

1. Nahtmaterial-Handhabungsvorrichtung für einen insbesondere aus einem chirurgischen Nahtmaterial realisierten Faden (60) mit einem Nähkopf aufweisend einen ersten (10) und einen zweiten (20) Backenabschnitt, die relativ zueinander bewegbar, insbesondere schwenk- und/oder klappbar, ausgebildet sind,
und den Backenabschnitten zugeordneten ersten Nadelmitteln (50), die zum Durchführen eines ersten (D1) Durchstich- sowie Fadenmitnahmevorgangs eines ersten Fadenendes (63) des Fadens in einer geschlossenen und/oder zueinander bewegten Relativposition der Backenabschnitte oder durch das Bewegen mindestens eines der Backenabschnitte durch bevorzugt manuelle Betätigung bewegbar ausgebildet sind,
sowie mit dem Nähkopf, insbesondere den Backenabschnitten, zugeordnete, gesondert von den ersten Nadelmitteln (50) betätigbare und diesen benachbart vorgesehene zweite Nadelmittel (30), die zum Durchführen eines zweiten (D2, D3) Durchstich- sowie Fadenmitnahmevorgangs für das dem ersten Fadenende entgegengesetze zweite Fadenende (40) des Fadens (60) in der geschlossenen bzw. zueinander bewegten Relativposition der Backenabschnitte ausgebildet sind,
wobei die zweiten Nadelmittel zum Durchführen eines lokal beabstandeten und aufeinanderfolgenden zweifachen Durchstechens (D2, D3) eines in der geschlossenen bzw. zueinander bewegten Relativposition zwischen den Backenabschnitten einlagig aufgenommenen Nahtträgers (110) einen zum Ausführen einer das zweite Fadenende (40) mitnehmenden Schwenk- und/oder Bogenbewegung ausgebildeten Mitnehmerabschnitt (31) aufweisen.

2. Vorrichtung nach Anspruch 1, **dadurch gekennzeichnet, dass** die zweiten Nadelmittel einen bevorzugt kreisbogenförmig gebogenen und endseitig den Mitnehmerabschnitt zum lösbaren Einsetzen des zweiten Fadenendes (40) ausgebildeten Bogenschaft (31) aufweisen, wobei der Mitnehmerabschnitt bevorzugt nadelartig ausgebildet ist oder eine Nadel (41) tragen kann.

3. Vorrichtung nach Anspruch 1 oder 2, **dadurch gekennzeichnet, dass** die zweiten Nadelmittel eine motorisch und/oder manuell drehbar gelagerte Schaftbaugruppe (32, 33) aufweisen, die weiter bevorzugt an einer der Backenabschnitte drehbar festgelegt ist.

4. Vorrichtung nach Anspruch 3, **dadurch gekennzeichnet, dass** die Schaftbaugruppe an einem Übergangsabschnitt (32) zum Mitnehmerabschnitt exzentrisch ausgelenkt und/oder gekröpft ausgebildet ist.

5. Vorrichtung nach einem der Ansprüche 3 oder 4, **dadurch gekennzeichnet, dass** an einem der Backenabschnitte der Schaftbaugruppe benachbart ein Führungs- und/oder Aufnahmeabschnitt (11, 12, 17) für das erste Fadenende (63) zum Zusammenwirken mit den ersten Nadelmitteln (50) ausgebildet ist.

6. Vorrichtung nach einem der Ansprüche 1 bis 5, **dadurch gekennzeichnet, dass** einem endseitigen Nadelabschnitt (41) der zweiten Nadelmittel ein diesen gleitend führender Gleitabschnitt (21) an einem gegenüberliegenden der Backenabschnitte zugeordnet ist.

7. Vorrichtung nach einem der Ansprüche 1 bis 6, **dadurch gekennzeichnet, dass** mindestens einem der Backenabschnitte Fadenendeaufnehmermittel (80) zugeordnet sind, die so ausgebildet sind, dass darin das zweite Fadenende nach dem zweifachen Durchstechen, bevorzugt rastend oder schnappend, eingeführt und vom Mitnehmerabschnitt (31) der zweiten Nadelmittel (30) gelöst werden kann.

## Claims

1. A suture material handling device for a thread (60) made in particular from a surgical suture material comprising a suturing head having a first (10) and a second (20) jaw section, which are designed to be capable of being moved relative to one another, in particular pivoted and/or folded,
and first needle elements (50), which are associated with the jaw sections, and which are designed for performing a first (D1) piercing as well as thread entraining operation of a first thread end (63) of the thread in a relative position that is closed and/or in which the jaw sections have been moved toward one another or are designed to be movable by the movement of at least one of the jaw sections, by preferably manual actuation,
and
second needle elements (30), which are associated with the suturing head, in particular with the jaw sections, and which can be actuated separately from the first needle elements (50) and which are provided adjacent to the first needle elements and which are designed to perform a second (D2, D3) piercing as well as thread entraining operation for the second thread end (40), which is opposite the first thread end, of the thread (60) in the relative position of the jaw sections that is closed or in which the jaw sections have been moved toward one another, respectively,
wherein, in order to perform a locally spaced apart and consecutive double piercing (D2, D3) of a suture carrier (110), which is received in a single layer between the jaw sections in the relative position that is closed or in which the jaw sections have been moved toward one another, respectively, the second needle elements have an entraining section (31), which is designed to perform a pivoting and/or curved motion that entrains the second thread end (40).

2. The device according to claim 1, **characterized in that** the second needle elements have a curve shank (31), which is preferably arcuately curved and which embodies the entraining section on the end side for releasably inserting the second thread end (40), wherein the entraining section is preferably designed in a needle-like manner or can carry a needle (41).

3. The device according to claim 1 or 2, **characterized in that** the second needle elements have a shank assembly (32, 33), which is rotatably mounted by way of motor and/or manually and which more preferably is rotatably secured to one of the jaw sections.

4. The device according to claim 3, **characterized in that** the shank assembly is designed so as to be deflected or cropped, respectively, eccentrically to the entraining section at a transition section (32).

5. The device according to one of claims 3 or 4, **characterized in that** a guide and/or receiving section (11, 12, 17) for the first thread end (63) is formed adjacent to one of the jaw sections of the shank assembly for interacting with the first needle elements (50).

6. The device according to one of claims 1 to 5, **characterized in that** a sliding section (21) guiding an end-side needle section (41) of the second needle elements in a sliding manner is associated therewith on an opposite one of the jaw sections.

7. The device according to one of claims 1 to 6, **characterized in that** thread end receiver elements (80), which are designed such that the second thread end can be inserted therein after the double piercing, preferably in a latching or snapping manner, and can be released from the entraining section (31) of the second needle elements (30), are associated with at least one of the jaw sections.

## Revendications

1. Dispositif de manipulation de suture pour un fil (60), notamment formé de suture chirurgicale, ayant
une tête de couture comprenant une première (10) et une seconde (20) partie de mâchoire étant réalisées de manière mobile l'une par rapport à l'autre, notamment de manière pivotante et/ou rabattable, et ayant des premiers moyens d'aiguille (50) étant assignés aux parties de mâchoire et étant configurés afin de réaliser une première (D1) opération de perçage et de transport du fil d'une première extrémité de fil (63) du fil dans une position relative fermée et/ou déplacée l'une par rapport à l'autre des parties de mâchoire ou étant réalisées de manière mobile par le déplacement d'au moins une des parties de mâchoire, de préférence par un actionnement manuel,
et ayant
des deuxièmes moyens d'aiguille (30) étant assignés à la tête de couture, notamment aux parties de mâchoire et pouvant être actionnés indépendamment des premiers moyens d'aiguille (50) et étant prévus adjacents auxdits moyens et étant configurés afin de réaliser une deuxième (D2, D3) opération de perçage et de transport du fil pour la deuxième extrémité de fil (40) du fil (60) opposée à la première extrémité de fil dans la position relative fermée et/ou déplacée l'une par rapport à l'autre des parties de mâchoire,
les deuxièmes moyens d'aiguille ayant une partie d'entraînement (31) étant configurée afin d'effectuer un mouvement de pivotement et/ou en arc en transportant la deuxième extrémité de fil (40) pour réaliser un double perçage (D2, D3) successif étant espacé localement d'un support de couture (110) étant logé en une seule couche entre les parties de mâchoire dans la position relative fermée ou déplacée l'une par rapport à l'autre.

2. Dispositif selon la revendication 1, **caractérisé en ce que** les deuxièmes moyens d'aiguille ont une tige d'arc (31) qui est courbée, de préférence en arc de cercle, et qui réalise la partie d'entraînement à l'extrémité pour l'insertion amovible de la deuxième extrémité de fil (40), la partie d'entraînement étant réalisée, de préférence, en forme d'aiguille ou étant capable de porter une aiguille (41).

3. Dispositif selon la revendication 1 ou la revendication 2, **caractérisé en ce que** les deuxièmes moyens d'aiguille ont un assemblage de tige (32, 33) qui est monté de manière rotative par un moteur et/ou manuellement et qui est fixé, plus préférentiellement, sur une des parties de mâchoire.

4. Dispositif selon la revendication 3, **caractérisé en ce que** l'assemblage de tige est réalisé à une partie de transition (32) et dévié et/ou coudé de manière excentrique par rapport à la partie d'entraînement.

5. Dispositif selon la revendication 3 ou la revendication 4, **caractérisé en ce qu'**une partie de guidage et/ou de logement (11, 12, 17) pour la première extrémité de fil (63) est réalisée de manière adjacente à une des parties de mâchoire de l'assemblage de tige afin d'interagir avec les premiers moyens d'aiguille (50).

6. Dispositif selon l'une quelconque des revendications 1 à 5, **caractérisé en ce qu'**une partie coulissante (21) qui guide une partie d'aiguille (41) d'extrémité de manière coulissante est assignée à ladite partie d'aiguille des deuxièmes moyens d'aiguille sur une partie opposée des parties de mâchoire.

7. Dispositif selon l'une quelconque des revendications 1 à 6, **caractérisé en ce qu'**au moins une des parties de mâchoire est assignée des moyens de logement de l'extrémité de fil (80) qui sont configurés de telle manière que la deuxième extrémité de fil peut être insérée, de préférence de manière encliquetée ou enclenché, dans lesdits moyens après le double perçage et que ladite extrémité de fil peut être détachée de la partie d'entraînement (31) des deuxièmes moyens d'aiguille (30).
